(19) Europäisches Patentamt
European Patent Office
Office européen des brevets

(11) **EP 4 740 839 A1**

(12) **EUROPEAN PATENT APPLICATION**

(43) Date of publication:
**13.05.2026  Bulletin 2026/20**

(51) International Patent Classification (IPC):
**A61B 5/00** $^{(2006.01)}$

(21) Application number: **24211081.5**

(22) Date of filing: **06.11.2024**

(52) Cooperative Patent Classification (CPC):
**A61B 5/742;** A61B 5/339

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Koninklijke Philips N.V.**
**5656 AG Eindhoven (NL)**

(72) Inventors:
• **MENKEN, Jonas**
  **Eindhoven (NL)**
• **TESSEL, Uli**
  **Eindhoven (NL)**
• **HUIJBREGTS, Laurentia Johanna**
  **5656AG Eindhoven (NL)**
• **VAN LIESHOUT, Ron Martinus Laurentius**
  **Eindhoven (NL)**

(74) Representative: **Philips Intellectual Property & Standards**
**High Tech Campus 52**
**5656 AG Eindhoven (NL)**

(54) **CONTROLLING DISPLAY OF DATA**

(57)     Proposed concepts aim to provide schemes, solutions, concepts, designs, methods and systems pertaining to a method for controlling a display of data. In particular, embodiments aim to provide a method for controlling a display, in which the evolution of the display position of a second eraser bar described by a second display control signal is controlled based on the evolution in the display position of a first eraser bar described by a first display control signal. This may, for example, help to reduce disturbance of waveform display when adding or removing a window.

FIG. 2A

FIG. 2B

FIG. 2C

**EP 4 740 839 A1**

**Description**

FIELD OF THE INVENTION

This invention relates to the field of subject monitoring, and more specifically, to the field of the controlling the display of waveforms in subject monitoring displays.

BACKGROUND OF THE INVENTION

Subject monitoring displays may be used in clinical practice to provide a visual representation of subject (e.g. patient) health data to a clinician and thereby aid with subject monitoring and diagnosis. Such displays typically include waveforms representing measured time-varying physiological signals such as photoplethysmography (PPG) signals, ECG signals, and arterial blood pressure (ABP) signals. Typically, these waveforms are drawn on a subject monitoring display in real time, with the waveform being updated with the latest monitoring data as and when it becomes available.

Subject monitoring displays may also display other information including windows such as menus, trendlines, and values of physiological parameters. These windows may be overlayed on top of any waveforms displayed on the screen or may be non-overlay windows that reduce the area available for waveform display (i.e. do not overlay or overlap displayed waveforms). When such windows are added to or deleted from the display, the display of waveforms can be altered to accommodate the addition/deletion of the window. In such cases, real-time monitoring of the subject may be interrupted by the alteration in the display, potentially leading to a discontinuity in a clinician's assessment of the subject and resulting in a decreased quality of care.

SUMMARY OF THE INVENTION

The invention is defined by the claims.

According to examples in accordance with an aspect of the invention, there is provided a computer implemented method for controlling a display of data.

The method comprises: generating a first display control signal for controlling a display in a first time interval, the first display control signal describing: the display of a waveform in a display area of the display, the waveform visually indicating the variation in the value of a time-varying signal over a first time period; and the display of a first eraser bar in the display area, the display position of the first eraser bar in the display area evolving over time to indicate the evolution in a time origin of the first time period with time such that at any given time the difference between the display position of the first eraser bar and the display position of a point in the waveform indicates the difference between the time origin of the first time period and a capture time associated with the point in the waveform; and generating a second display control signal for controlling the display in a second time interval that does not overlap with the first time interval, the second display control signal describing: the display of a window in a first portion of the display area, the display area further comprising a second portion in which the window is not displayed; the display of a truncated version of the waveform in the second portion of the display area, wherein the truncated version of the waveform visually indicates the variation in the time-varying signal over a second time period that is shorter than the first time period and at least partially overlaps with the first time period; and the display of a second eraser bar in the second portion of the display area, the display position of the second eraser bar in the second portion of the display area evolving over time to indicate the evolution in a time origin of the second time period with time such that at any given time the difference between the display position of the second eraser bar and the display position of a point in the truncated version of the waveform indicates the difference between the time origin of the second time period and a capture time associated with the point in the truncated version of the waveform, wherein the evolution in the position of the second eraser bar over time is controlled based on the evolution of the display position of the first eraser bar over time.

Proposed concepts thus aim to provide schemes, solutions, concepts, designs, methods and systems pertaining to a method for controlling a display of data. In particular, embodiments aim to provide a method for controlling a display, in which the evolution of the display position of a second eraser bar described by a second display control signal is controlled based on the evolution in the display position of a first eraser bar described by a first display control signal. This may, for example, help to reduce disturbance of waveform display when adding or removing a window.

Embodiments involve the insertion/addition of a non-overlay window into a display of a waveform and a subsequent modification to the display of the waveform that may allow for continuous monitoring of a time-varying signal. The second display control signal of the present invention describes the display of a window in a first portion of the display area and the display of a truncated version of a waveform in a second portion of the display area in which the window is not displayed. This ensures that no portion of the waveform is "hidden" behind the display of the non-overlay window, thus permitting the value of the time-varying signal to be continuously displayed to the clinician. The truncated version of the waveform describes the value of the signal over a shorter second time period than the time period described by the waveform before the window is inserted. This ensures that time scale of the waveform is not scaled down when the space available for its display decreases due to the insertion of the window - for clinicians to be able to interpret the signal, it is preferable that the scale stays the same. Instead, to address the issue of a reduced area being available for display, a shorter portion of historic data of the time-varying signal is displayed in the truncated version of the waveform. This may be beneficial for accurate visual interpretation of the waveform by a clinician in real time.

In the proposed display concept(s), the evolution in the display position of a second eraser bar described by the second display control signal is controlled based on the evolution in the display position of a first eraser bar described by the first display control signal. The term 'eraser bar' is widely used in the context of waveform display and used to describe a point in the waveform that describes the most recently available data which moves across the display to indicate the passage of time. Thus, the eraser bar describes a point in the waveform at the intersection of new data being inserted and old data being displayed. Often, the point itself is devoid of display data and thus appears to represent a moving eraser of displayed data that wipes away old data (for it to be replaced with new data).

By controlling the evolution/change in the display position of the second eraser bar once the window has been inserted (based on the evolution in the display position of the first eraser bar over time before the window is inserted), the proposed method may facilitate continuous monitoring of the newly-available data for the signal even when a window is added or removed to/from a display.

Ultimately, therefore, an improved method for displaying a waveform describing a time-varying signal may be provided by the proposed concept(s).

In some embodiments, the second display control signal may further describe the display of a window entering the first portion of the display area at a finite window entry speed over a window entry time period within the second time interval, and the evolution of the display position of the second eraser bar over time may be further controlled based on the value of the window entry speed. Gradual insertion of the window into the display may result in improved monitoring of the time-varying signal due to the smoother transition between the display of the waveform and the display of the truncated version of the waveform. Controlling the evolution in the display position of the second eraser bar over time based on the value of the window entry speed may help to ensure that the second eraser bar is always clearly visible.

In some embodiments, controlling the evolution in the display position of the second eraser bar over time may comprise controlling a speed at which the second eraser bar moves across the display over the window entry time period to be equal to the sum of the speed of the first eraser bar at the end of the first time interval and an additional speed, and the value of the additional speed may be determined based on the value of the window entry speed and the display position of the second eraser bar. This may ensure that the second eraser bar moves continuously across the display even during the insertion of the window in such a way that the second eraser bar is always clearly visible. This may aid in visual interpretation of the truncated version of the waveform during the window entry period.

In some embodiments, the additional speed may be equal to a scale factor multiplied by the window entry speed, the scale factor being greater than or equal to zero and less than or equal to one ("1"), and the value of the scale factor may vary as a function of the display position of the second eraser bar. This may ensure that the second eraser bar is not obscured by the display of the window and moves across the display in an intuitive manner.

In some embodiments, the second display control signal may control the window entry speed of the window based on at least one of: a display position of the first eraser bar at the end of the first time interval; and a speed at which the display position of the first eraser bar changes over time at the end of the first time interval. This provides an alternative or additional way to control the relative display positions of the window and the second eraser bar to permit accurate and real-time monitoring of the value of the time-varying signal.

In some embodiments, controlling the evolution in the display position of the second eraser bar over time may comprise controlling an initial display position of the second eraser bar at the start of the second time interval comprising: controlling the initial display position of the second eraser bar to have a first value if the display position of the first eraser bar at the end of the first time interval is in the first portion of the display area; and controlling the initial display position of the first eraser bar to have a second value if the display position of the second eraser bar at the end of the first time interval is in the second portion of the display area. Thus, the properties of the first portion of the display area in which the window is displayed may be used to inform the control of the second eraser bar resulting in more accurate control of waveform display to account for the inclusion or deletion of a window.

In some embodiments, controlling the initial display position of the second eraser bar to have a second value may comprise controlling the initial display position of the second eraser bar to be the same as the display position of the first eraser bar at the end of the first time interval. In this way, continuity in the display position of the eraser bar can be achieved across the first and second time intervals.

According to another aspect of the present invention there is provided another method for controlling a display of data. The method comprises: generating a first display control signal for controlling a display in a first time interval, the first display control signal describing: the display of a window in a first portion of a display area, the display area further comprising a second portion in which the window is not displayed; the display of a waveform in the second portion of the display area, the waveform visually indicating the variation in a time-varying signal over a first time period; and the display of a first eraser bar in the second portion of the display area, the display position of the first eraser bar in the second portion of the display area evolving over time to indicate the evolution in a time origin of the first time period with time such that at any given time the difference between the display position of the first eraser bar and the display position of a point in the waveform indicates the difference between the time origin of the first time period and a capture time associated with the point in the waveform; and generating a second display control signal for controlling a display in a second time interval that does not overlap with the first time interval, the second display control signal describing: the display of an elongated version of the waveform in the display area, wherein the elongated version of the waveform visually indicates the variation in the time-varying signal over a second time period that is longer than the first time period and at least partially overlaps with the first time period; and the display of a second eraser bar in the display area, the display position of the second eraser bar in the display area evolving over time to indicate the evolution in a time origin of the second time period with time such that at any given time the difference between the display position of the second eraser bar and the display position of a point in the elongated version of the waveform indicates the difference between the time origin of the second time period and a capture time associated with the point in the elongated version of the waveform, wherein the evolution of the position of the second eraser bar over time is controlled based on the evolution of the display position of the first eraser bar over time. Thus, the present invention also involves the continuous display of a waveform describing a time-varying signal when a window is removed from a display.

In some embodiments of this method, the first display control signal may further describe the display of the window retracting from the first portion of the display area at a finite window exit speed over a window exit time period within the first time interval, and the evolution of the display position of the first eraser bar during the window exit time period may be controlled based on the value of the window exit speed. Thus, in a similar manner to that discussed above for window insertion, the display of the window may be removed gradually to result in a smooth transition between the display of the waveform and the display of the elongated version of the waveform.

In some embodiments, controlling the evolution of the display position of the first eraser bar during the window exit time period may comprise controlling the speed at which the display position of the first eraser bar changes over time based on the value of the window exit speed and the display position of the first eraser bar. This advantageously permits continuous variation in the display position of the first eraser bar for reliable waveform interpretation.

In some embodiments of any of the methods described above, controlling the evolution in the display position of the second eraser bar over time may comprise controlling the evolution in the display position of the second eraser bar based on at least one of: a display position of the first eraser bar at the end of the first time interval; and a speed at which the display position of the first eraser bar changes over time at the end of the first time interval. These aspects of the evolution of the display position of the first eraser bar may be particularly important to achieve continuity between the displays described by the first and second display control signals.

In some embodiments of any of the methods described above, controlling the evolution in the display position of the second eraser bar over time may comprise, controlling at least one of: an initial display position of the second eraser bar at the start of the second time interval; and an initial speed at which the display position of the second eraser bar changes over time at the start of the second time interval. These aspects of the display of the second eraser bar may also have significant impact on permitting continuous interpretation of the waveform across the first and second time intervals.

According to another aspect of the present invention, there is provided a computer program comprising computer program code means adapted, when said computer program is run on a computer, to implement the method of any of the above-described embodiments.

According to yet another aspect there is provided a processing arrangement for controlling a display of data configured to run the computer program described above. This processing arrangement may be integrated into a display system further comprising a display controlled by the processing arrangement.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 is a simplified flow diagram of a method for controlling a display of data according to a proposed embodiment;
Fig. 2A is an illustration of a display controlled by a first display control signal according to embodiments of the proposed invention;
Fig. 2B and 2C are illustrations of a display controlled by a second display control signal according to embodiments of the proposed invention;
Fig. 3 is a simplified flow diagram of a method for controlling a display of data according to another aspect of the present invention;

Fig. 4A is an illustration of a display controlled by a first display control signal according to a proposed embodiment;

Fig. 4B is an illustration of a display controlled by a second display control signal according to a proposed embodiment;

Fig. 5 is a simplified block diagram of display system for displaying data according to an aspect of the present invention; and

Fig. 6 illustrates an example of a computer within which one or more parts of an embodiment may be employed.

DETAILED DESCRIPTION OF THE EMBODIMENTS

The invention will be described with reference to the Figures.

It should be understood that the detailed description and specific examples, while indicating exemplary embodiments of the apparatus, systems, and methods, are intended for purposes of illustration only and are not intended to limit the scope of the invention. These and other features, aspects, and advantages of the apparatus, systems, and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Variations to the disclosed embodiments can be understood and effected by those skilled in the art of practicing the claimed invention, from a study of the drawings, the disclosure, and the appended claims. In the claims, the word "comprising" does not exclude other elements or steps, and the indefinite article "a" or "an" does not exclude a plurality.

Implementations in accordance with the present disclosure relate to various techniques, methods, schemes and/or solutions pertaining to controlling a display. According to proposed concepts, a number of possible solutions may be implemented separately or jointly. That is, although these possible solutions may be described below separately, two or more of these possible solutions may be implemented in one combination or another.

Embodiments of the invention aim to provide a concept for controlling a display to display a waveform and to manage the display of the waveform when a non-overlay window is added or removed from the display. In particular it is proposed to control a change in the display position of an eraser bar in manner that helps to reduce disturbance of the displayed waveform when the window is added/removed. This may provide for a smooth display transition, thus enabling a viewer to maintain sight/awareness of the waveform information.

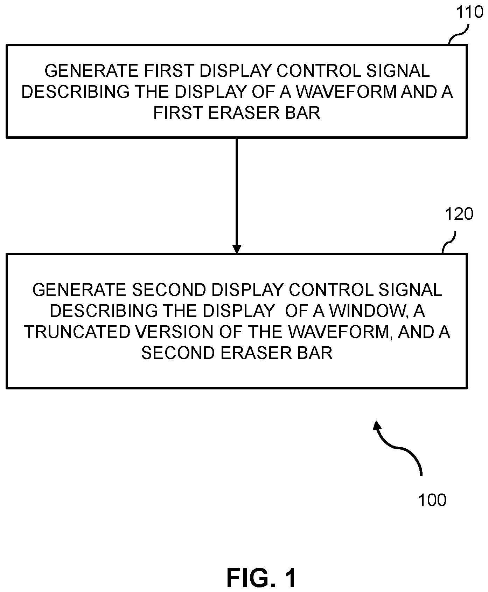

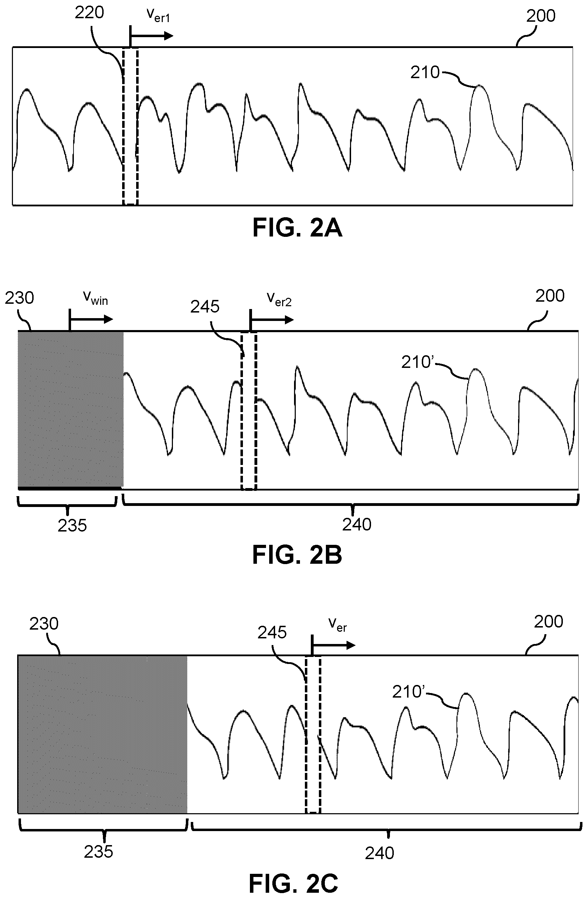

Referring now to Fig. 1, there is depicted a simplified flow diagram of a method 100 for controlling a display of data according to an exemplary embodiment. Also, for context of describing the embodiment of Fig. 1, Figs. 2A-2C are provided as illustrations of a display controlled by first and second display control signals according to the embodiment of Fig. 1. Specifically, Fig. 2A is an illustration of a display controlled by a first display control signal according to the embodiment of Fig. 1. Fig. 2B then illustrates the display controlled by a second display control signal according to a first example of the embodiment of Fig. 1, whereas Fig. 2B illustrates the display controlled by a second display control signal according to a second example of the embodiment of Fig. 1.

The method 100 comprises the step 110 generating a first display control signal for controlling a display in a first time interval.

As depicted in Fig. 2A, the first display control signal describes the display of a waveform 210 in a display area 200 of the display. The waveform 210 visually indicates the variation in the value of a time-varying signal over a first time period. The first display control signal also describes the display of a first eraser bar 220 in the display area 200.

The display position of the first eraser bar 220 in the display area 200 evolves over time to indicate the evolution in a time origin of the first time period with time. The first eraser bar 220 thus moves across the display area 200 (from left to right) at a first speed $V_{er1}$ (as indicated by the arrow labelled $V_{er1}$ in Fig. 2A).

In this way, the first eraser bar 220 moves such that, at any given time, the difference between the display position of the first eraser bar 220 and the display position of a point in the waveform 210 indicates the difference between the time origin of the first time period and a capture time associated with the point in the waveform.

Responsive to the requested addition of a non-overlay window to the display area 200, the method proceeds to step 120. In step 120, the method generates a second display control signal for controlling the display in a second time interval that does not overlap with the first time interval.

As depicted in Figs. 2B & 2C, the second display control signal describes the display of a window 230 in a first portion 235 of the display area 200, such that the display area further comprises a second portion 240 in which the window 230 is not displayed. In the example of Fig. 2C, the first portion 235 of the display area 200 is larger than the first portion 235 of the display area 200 in Fig. 2B. Fig. 2B and 2C therefore demonstrate how the differing size of the window 230 impacts the size of the second portion 240 of the display area 200 in which a truncated version of the waveform 210' is displayed after addition/insertion of the window 230.

The second display control signal also describes the display of a truncated version of the waveform 210' in the second portion 240 of the display area 200. Here, the truncated version of the waveform 210' visually indicates the variation in the time-varying signal over a second time period that is shorter than the first time period and at least partially overlaps with the first time period

The second display control signal yet further describes the display of a second eraser bar 245 in the second portion 240 of the display area 200.

The display position of the second eraser bar 245 in the second portion 240 of the display area 200 evolves over time to indicate the evolution in a time origin of the second time period with time. The second eraser bar 245 thus moves across the display area 200 (from left to right) at a second speed $V_{er2}$ (as indicated by the arrow labelled $V_{er2}$ in Fig. 2B).

In this way, the second eraser bar 245 moves such that, at any given time, the difference between the display position of the second eraser bar 245 and the display position of a point in the truncated version of the waveform 210' indicates the difference between the time origin of the second time period and a capture time associated with the point in the truncated version of the waveform.

Importantly, however, the evolution in the position of the second eraser bar 245 over time is controlled based on the evolution of the display position of the first eraser bar 220 over time. In this way, disturbance of waveform display is reduced/avoided when adding the window 230 to the display area 200.

Furthermore, in the embodiment of Fig. 1, the second display control signal further describes the display of the window 230 entering the first portion 235 of the display area 200 at a finite window entry speed $V_{win}$ over a window entry time period within the second time interval (as depicted by the arrow labelled $V_{win}$ in Fig. 2B). To account for this transitional display of the window 230, the evolution of the display position of the second eraser bar 245 over time is further controlled based on the value of the window entry speed $V_{win}$. Gradual insertion of the window 230 into the display area 200 may result in improved monitoring of the time-varying signal, due to providing a smoother transition between the display of the waveform 210 and the display of the truncated version of the waveform 210'. Also, controlling the evolution in the display position of the second eraser bar 245 over time based on the value of the window entry speed $V_{win}$ may help to ensure that the second eraser bar 245 is always clearly visible.

Purely by way of example, in some embodiments, the second display control signal may control the window entry speed $V_{win}$ of the window 230 based on at least one of: a display position of the first eraser bar 220 at the end of the first time interval; and a speed $V_{er1}$ at which the display position of the first eraser bar 220 changes over time at the end of the first time interval. This example may provide an alternative or additional way to control the relative display positions of the window 230 and the second eraser bar 245 so as to permit accurate and real-time monitoring of the value of the time-varying signal.

Yet further, in the embodiment of Fig. 1, controlling the evolution in the display position of the second eraser bar 245 over time also comprises controlling the second speed $V_{er2}$ at which the second eraser bar moves across the display area 200 over the window entry time period to be equal to the sum of the first speed $V_{er1}$ of the first eraser bar 220 at the end of the first time interval and an additional speed. Here, the value of the additional speed is determined based on the value of the window entry speed $V_{win}$ and the display position of the second eraser bar 245. This may ensure that the second eraser bar 245 moves continuously across the display even during the insertion of the window 230 in such a way that the second eraser bar 245 is always clearly visible. This may, for example, assist visual interpretation of the truncated version of the waveform 210' during the window entry period.

By way of example, in one implementation, the additional speed may be equal to a scale factor multiplied by the window entry speed $V_{win}$, the scale factor being greater than or equal to zero and less than or equal to one ("1"). The value of the scale factor may, for example, vary as a function of the display position of the second eraser bar 245. This may ensure that the second eraser bar 245 is not obscured by the display of the window 230 and moves across the display in an intuitive manner.

By way of example, in one implementation, controlling the evolution in the display position of the second eraser bar 245 over time may comprise, controlling at least one of: an initial display position of the second eraser bar 245 at the start of the second time interval; and an initial speed at which the display position of the second eraser bar 245 changes over time at the start of the second time interval.

Also, by way of further example, controlling the initial display position of the second eraser bar 245 may comprise: controlling the initial display position of the second eraser bar 245 to have a first value if the display position of the first eraser bar 220 at the end of the first time interval is in the first portion of the display area; and controlling the initial display position of the first eraser bar 220 to have a second value if the display position of the second eraser bar at the end of the first time interval is in the second portion of the display area. In this way, the properties of the first portion 230 of the display area 400 in which the window is displayed may be used to inform the control of the second eraser bar 245 resulting in more accurate control of waveform display to account for the inclusion or deletion of a window.

From the above description, the embodiment described with reference to Figs. 1 & 2A-2C involves the insertion of a non-overlay window 230 into a display 200 of a waveform 210 and a subsequent modification to the display of the waveform 210 that may allow for continuous monitoring of a time-varying signal. The second display control signal describes the display of the window 230 in a first portion 235 of the display area 200 and the display of a truncated version of a waveform 210' in a second portion 240 of the display area 200 in which the window 230 is not displayed. This ensures that no portion of the waveform 210 is "hidden" behind the display of the non-overlay window 230, thus permitting the value of the time-varying signal to be continuously displayed to the clinician.

The truncated version of the waveform 210' describes the value of the signal over a shorter second time period than the time period described by the waveform before the window is inserted. This ensures that time scale of the waveform is not significantly scaled down when the space available for its display decreases due to the insertion of the window 230. Rather, to address the issue of a reduced area being available for display, a shorter portion of historic data of the time-varying signal is displayed in the truncated version of the waveform 210'. This assists accurate visual interpretation of the waveform by a clinician in real time.

As can be seen from Fig 2A-2C, this truncation of the waveform has the further effect that the waveform displayed on either side of the eraser bar appears to move over time. In contrast, during normal display of the waveform, when there is no window displayed, the waveform may appear static. This means that between each successive pass of the eraser bar, each point on the display displays the same point in the waveform. Once the point is "overtaken" by the eraser bar, the historic waveform data is replaced by new waveform data which is then continuously displayed at this point until the eraser bar passes over the point again. However, during the window entry period this will not necessarily be the case. Once the window enters the display, the display shows a truncated version of the waveform that shows a shorter portion of historic data of the time-varying signal. Therefore, there is a recalculation of the portion of the historic waveform displayed based on the width of the display available for display of the waveform. This results in the waveform appearing to move across the display as the display position of the eraser bar and the window evolves over time. This accounts for the apparent motion of the waveform seen in Fig, 2A-2C.

Although Fig. 1 and Fig. 2 only relate to controlling the evolution of the position of the second eraser bar during the window entry period, it is envisaged that this control may extend after the window has fully entered the display. For example, in certain instances the control of the display position of the second eraser bar may continue until the end of the current refresh period. In other words, until the second eraser bar is displayed at the edge of the second portion of the display area. This may assist in providing a smooth transition between the displays.

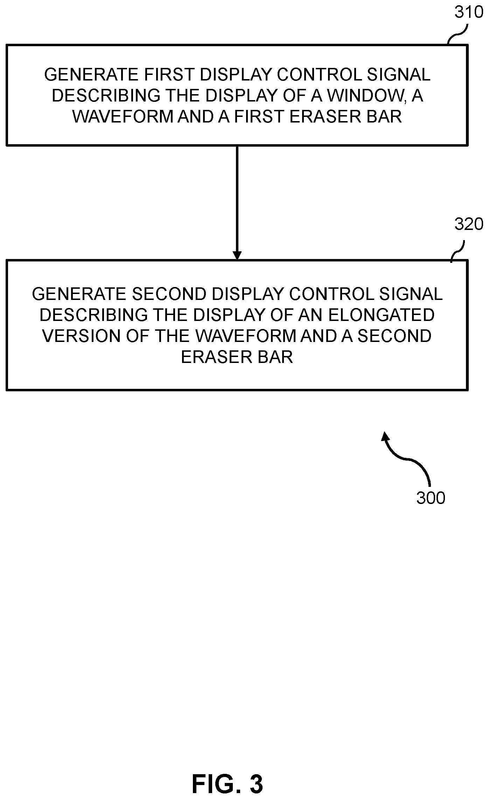

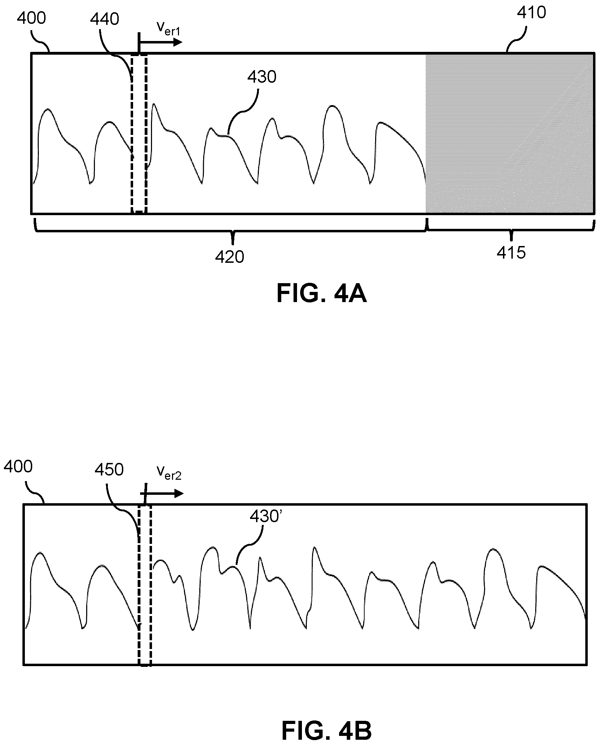

Referring now to Fig. 3, there is depicted a simplified flow diagram of a method 300 for controlling a display of data according to another exemplary embodiment. Also, for context of describing the embodiment of Fig. 3, Figs. 4A-4B are provided as illustrations of a display controlled by first and second display control signals according to the embodiment of Fig. 3. Specifically, Fig. 4A is an illustration of a display controlled by a first display control signal according to the embodiment of Fig. 3. Fig. 4B then illustrates the display controlled by a second display control signal according to an example of the embodiment of Fig. 3.

The method 300 comprises the step 310 of generating a first display control signal for controlling a display in a first time interval.

As depicted in Fig. 4A, the first display control signal describes the display of a window 410 in a first portion 415 of a display area 400. The display area further comprises a second portion 420 in which the window 410 is not displayed.

The first display control signal also describes the display of a waveform 430 in the second portion 420 of the display area 400. The waveform visually indicates the variation in a time-varying signal over a first time period.

The first display control signal yet further describes the display of a first eraser bar 440 in the second portion 420 of the display area 400. The display position of the first eraser bar 440 in the second portion 420 of the display area 400 evolves over time to indicate the evolution in a time origin of the first time period with time. The first eraser bar 440 thus moves across the display area 400 (from left to right) at a first speed $V_{er1}$ (as indicated by the arrow labelled $V_{er1}$ in Fig. 4A).

In this way, the first eraser bar 440 moves such that, at any given time, the difference between the display position of the first eraser bar 440 and the display position of a point in the waveform indicates the difference between the time origin of the first time period and a capture time associated with the point in the waveform.

Responsive to the requested deletion/removal of the window 410 from the display area 400, the method proceeds to step 320. In step 320, the method generates a second display control signal for controlling the display in a second time interval that does not overlap with the first time interval.

As depicted in Fig. 4B, the second display control signal describes the display of an elongated version of the waveform 430' in the display area 400. Here, the elongated version of the waveform 430' visually indicates the variation in the time-varying signal over a second time period that is longer than the first time period and at least partially overlaps with the first time period.

The second display control signal also describes the display of a second eraser bar 450 in the display area 400.

The display position of the second eraser bar 450 in the display area 400 evolves over time to indicate the evolution in a time origin of the second time period with time. The second eraser bar 450 thus moves across the display area 400 (from left to right) at a second speed $V_{er2}$ (as indicated by the arrow labelled $V_{er2}$ in Fig. 4B).

In this way, the second eraser bar 450 moves such that, at any given time, the difference between the display position of the second eraser bar 450 and the display position of a point in the elongated version of the waveform 430' indicates the difference between the time origin of the second time period and a capture time associated with the point in the elongated version of the waveform.

Importantly, however, the evolution of the position of the second eraser bar 450 over time is controlled based on the evolution of the display position of the first eraser bar 440 over time. In this way, disturbance of waveform display is reduced/avoided when removing the window 410 from the display area 400.

Furthermore, in the embodiment of Fig. 3, the first display control signal further describes the display of the window 410 retracting from the first portion 415 of the display area 400 at a finite window exit speed over a window exit time period within the first time interval. To account for this transitional removal of the window 410, the evolution of the display position of the first eraser bar 440 during the window exit time period is further controlled based on the value of the window exit speed. Thus, in a similar manner to that discussed above for window insertion, the display of the window 410 may be removed gradually to result in a smooth transition between the display of the waveform 430 and the display of the elongated version of the waveform 430'.

Purely by way of example, in some embodiments, controlling the evolution of the display position of the first eraser bar 440 during the window exit time period comprises controlling the speed at which the display position of the first eraser bar 440 changes over time based on the value of the window exit speed and the display position of the first eraser bar 440. This advantageously permits continuous variation in the display position of the first eraser bar 440 for reliable waveform interpretation.

Yet further, in the embodiment of Fig. 3, controlling the evolution in the display position of the second eraser bar 450 over time comprises controlling the evolution in the display position of the second eraser bar based on at least one of: a display position of the first eraser bar 440 at the end of the first time interval; and a speed at which the display position of the first eraser bar 440 changes over time at the end of the first time interval. Such aspects of the evolution of the display position of the first eraser bar 440 may thus be accounted for to achieve continuity between the displays described by the first and second display control signals.

By way of example, in one implementation, controlling the evolution in the display position of the second eraser bar 450 over time may comprise, controlling at least one of: an initial display position of the second eraser bar 450 at the start of the second time interval; and an initial speed at which the display position of the second eraser bar 450 changes over time at the start of the second time interval.

From the above description, the embodiment described with reference to Figs. 3 & 4A-4B involves the removal of a non-overlay window 410 from a display 400 of a waveform 430 and a subsequent modification to the display of the waveform 430 that may allow for continuous monitoring of a time-varying signal. The second display control signal describes the display of an elongated version of a waveform 430' across the display area 400 after the window 410 is removed from the display area 400.

In some embodiments, controlling the initial display position of the second eraser bar to have a second value may comprise controlling the initial display position of the second eraser bar to be the same as the display position of the first eraser bar at the end of the first time interval. In this way, continuity in the display position of the eraser bar can be achieved across the first and second time intervals.

By way of further description and explanation of the proposed concept(s), exemplary embodiments will now be described in relation to subject monitoring. Such embodiments comprise:

(a) A display for showing at least one or more waveforms of physiological parameters and one or more other windows that can be added and removed, and, when these windows are displayed, they are covering an area that was otherwise covered by the waveforms, while, when the one or more windows are displayed, the waveforms will use a smaller area and the eraser bar, when it reaches the end of this smaller area, immediately starts at the beginning of this smaller area;

(b) A memory unit for storage of waveform data for at least the amount of time when they may be needed for display;

(c) A processor that calculates the place of the eraser bar during insertion or deletion of a window, where the eraser bar continuously has a place on the screen. In particular, in most cases, the processor determines $v_{extra}$ (see section "definitions" section below. In such a way, the clinician's following and interpretation of the waveforms gets least disturbed.

- Assumed Generalizations

The following examples are focused on subject monitors with subject (i.e. patient) data. However, the proposed concepts may be extended to any display showing a timeline with continuous (e.g. real-time) data and on which windows can be added or removed. Purely bay way of example, such data may relate to the weather, earth vibrations, water pressures, vehicle temperatures, etc.

Also, embodiments may not only relate to the addition and/or removal of a window from a display, but may also relate to when a displayed window's size, shape and/or orientation is changed.

For the avoidance of doubt, the following description of examples will employs the words 'left' and 'right'. These are based on conventional patient monitors that have waveforms running from left to right (and then starting over at the left when they reached the right edge of the display). It is to be understood that embodiments would also hold with left and right swapped when the waveform is running from right to left. Embodiments would also hold for other directions, like a waveform running from top to bottom. It is further assumes that the windows that are being added or removed get inserted or deleted in a direction that is in line with, or opposite to, the direction with which the eraser bar moves. If a window would be inserted or deleted in another direction, the proposed concepts would also hold, but the direction should be divided into a direction in line with (/opposite to) the direction with which the eraser bar moves and the direction perpendicular to it. The reasoning and equations described below would then hold for the direction in line with (/opposite to) the movement of the eraser bar.

Definitions

We will use the following definitions:

$t$ = time, with t=0 at the moment a window starts to get inserted or deleted
$T$ = time it takes to delete or insert the complete window.
$w$ = variable that reflects how far to the right of the screen a location is ($w = 0$ is completely at the left).
$w_{er}$ = location of the eraser bar along the w-axis.
$w_{er0}$ = location of the eraser bar along the w-axis at time t=0 (or right before the insertion/deletion of a window in case it gets inserted/deleted with infinite speed).
$W_{tot}$ = width available for waveforms when there is no additional window ('tot' stands for 'total').
$W_{win}$ = width of the window when it is on the screen in its final form.
$v_{er}$ = speed of the eraser bar.
$v_0$ = speed of the eraser bar when no windows are being inserted or deleted on the screen (i.e. its normal speed, being 25 mm/s for most waves (e.g. ECG, ABP, pleth) and 6.25 mm/s for slow waves like respiratory waves).
$v_{extra}$ = the extra speed that is given to the eraser bar when a window is being inserted/removed
$v_{win}$ = speed with which a window is inserted/deleted. When $v_{win} = \infty$, the window is placed on / removed from the screen at once.

Hence:

$Wt - W_{win}$ = width available for the waveforms when the additional window is completely on the screen.
$T = W_{win} / v_{win}$ (in case $v_{win}$ is constant)
$v_{er} = v_0 + v_{extra}$.

Note that, in general, there may be multiple waveforms on the screen. For the purpose of this description, focus is placed on only one waveform. The proposed concepts also hold for other displayed waveforms.

Eraser bar

Although 'eraser bar' is the term used for the place where the data is refreshed, it is noted that it does not necessarily need to work in the way that it adds and removes data at that place. Rather, the eraser bar is the place where the most recent data point is, while the previous data point is directly left to it, and the one previous to that one, directly left of that one, etc. It works like that up to the complete/far left of the available display space on the screen has been reached and then it starts from the complete right of the available display space on the screen, from where on the older data are displayed on the screen, back in time up to almost the eraser bar. As the system keeps all those datapoints that fit on the screen (and potentially more) in memory, the eraser bar can in principle be placed anywhere on the screen. Also, it is noted that the data that has already been plotted does not need to stay at the place where it has been plotted. In this way, it may be seen as a strip of data that may be pulled as a whole towards the right or pushed towards the left, for example.

Memory

As previously explained, it is undesirable to have the waveform scale change, when a window is added or removed to/from the display. According to the proposed concept(s), the duration of the data shown per unit length stays the same. Thus, when there is no window on the screen, the data shown on the screen have a duration of $W_{tot}/v_0$ (i.e. data from time $W_{tot}/v_0$ ago until the current time are shown). When there is a window on the screen, the data shown on the screen have a duration of $(W_{tot} - W_{win})/v_0$. However, when the window is removed, the display should be able to show, after removal, data from duration $W_{tot}/v_0$ again. It is therefore preferable for the memory to store the data needed to fill the screen, even though these data are not currently needed for displaying on the screen.

Note that it might not be the measurement datapoints themselves that the system keeps in memory. Instead, it might keep in memory the waveform that is drawn from it (e.g. a pixel-by-pixel trace), which is a processed form of the measurement data (including e.g. filtering and interpolation between datapoints).

Example Case I -Window entering from the left

Case: $v_{win} < \infty$

When a window is entering the display area from the left (with $v_{win} < \infty$), the animation may be made such that the window seems to push forward the eraser bar, i.e. $v_{extra} > 0$. Iit is preferable to do this when the eraser bar would get covered by the window if it would continue with its normal speed $v_0$. In that case, $v_{extra}$ should be sufficiently large for the eraser bar to end up at $w \geq W_{win}$ at time T (i.e. the eraser bar ends up at/past the right side of the window at the time the window has completely entered the display area).

When the eraser bar is at the complete/utmost left of the display area when the window starts to enter the screen, $v_{er}$ should at least be $v_{win}$. However, for the sake of giving the illusion that the window is pushing forward the eraser bar, $v_{er}$ should not be larger than $v_0 + v_{win}$. Hence, when the eraser bar is at the complete/utmost left of the display area when the window starts to enter the display area, $v_{win} - v_0 \leq v_{extra} \leq v_{win}$. This is under the assumption that $v_{win} \geq v_0$. In the unlikely case that $v_{win} < v_0$ (unlikely because the clinician does probably not want to wait long for a window to enter), the equation changes into $0 \leq v_{extra} \leq v_{win}$.

In a first exemplary embodiment, $v_{extra} = v_{win}$ (and thus $v_{er} = v_0 + v_{win}$) in the extreme case that the eraser bar is at the utmost of the display area when the window starts to enter.

On the other hand, it is typically preferred that the eraser bar is not pushed off the display area on the right side due to the entering of the window. Therefore, close to the right edge of the display area, $v_{extra}$ should be (near) zero. In particular, in the most extreme case, where at the end of time T (i.e. at the moment when the window has fully entered the display area), the eraser bar would exactly be at the right-hand side of the screen when it would just have moved with $v_{er} = v_0$, (i.e. the eraser bar was at $w = W_{tot} - T \cdot v_0$ at the moment the window started to enter the display area), there it would be chosen to have $v_{extra} = 0$.

For the eraser bar at widths in between the two extremes mentioned above, scaling can take place. We saw in the previous two paragraphs that in a first exemplary embodiment:

$v_{extra} = v_{win}$, for eraser bar at w=0 at time t=0
$v_{extra} = 0$, for eraser bar at $w = W_{tot} - T \cdot v_0$ at time t=0.

Then linear scaling gives that for the eraser bar at $w=w_{er0}$ at time t=0:

$$\text{vextra} = \text{vwin} \cdot (1 - (\text{wer0} / (\text{Wtot} - \text{T} \cdot \text{v0}))$$

(Here, linear scaling has been used, but in principle other monotonic functions can be used as well.)

The latter equation also holds for the two extremes. Thus, in an embodiment, this equation can be used for the $0 \leq w_{er0} \leq W_{tot} - T \cdot v_0$ at t=0.

When $W_{tot} - T \cdot v_0 < w_{er0} \leq W_{tot}$ at t=0, the eraser bar would reach beyond the right end of the display area already when it would move with $v_0$. In this case, in a preferred embodiment, $v_{extra}$ is chosen to be 0 until the eraser bar reaches the right end of the display area, and then, the eraser bar starts at the right-hand side of the window (at that point in time) and moves further with $v_{extra} = v_{win}$.

In the preceding embodiment, equations have been shown that can be used to calculate $v_{extra}$ for any location of the eraser bar at the moment the window starts to enter, in case $v_{win} < \infty$. However, when the equations of the preceding embodiment are used, in some occasions, it might still go wrong (in the sense that the window, while it gets inserted, catches up with the eraser bar and overtakes it). This is of course undesirable. This can happen when $v_{win}$ is large compared to $v_0$ and when $W_{win}$ is not substantially smaller than $W_{tot}$. To be precise: overtaking will take place when $v_{win} \cdot t > (W_{tot} - T \cdot v_0)(1 + v_0 \cdot t / w_{er0})$. Therefore, when $v_{win}$ is large compared to $v_0$, and when $W_{win}$ is not substantially smaller than $W_{tot}$, another embodiment should be used.

In a second exemplary embodiment, for any eraser bar in the area where the additional window will come, $v_{extra}$ is made equal to $v_{win}$, thus: $v_{extra} = v_{win}$, for $0 \leq w_{er0} \leq W_{win}$

Like in the first exemplary embodiment above:

$v_{extra} = 0$, for $w_{er0} = W_{tot} - T \cdot v_0$ and scaling takes place for the eraser bar at the places in between at t=0:

$$v_{\text{extra}} = v_{\text{win}}(W_{\text{tot}} - T \cdot v_0 - w_{\text{er0}})/(W_{\text{tot}} - T \cdot v_0 - W_{\text{win}}),$$

for $W_{\text{win}} \leq w_{\text{er0}} \leq W_{\text{tot}} - T \cdot v_0$, while for $W_{\text{tot}} - T \cdot v_0 < w_{\text{er0}} \leq W_{\text{tot}}$, $v_{\text{extra}} = 0$ until the eraser bar reaches the end of the display area, and then starts at the right side of the additional window with $v_{\text{extra}} = v_{\text{win}}$.

In a third exemplary embodiment, the eraser bar only gets 'pushed' by the window when it would otherwise be overtaken by the window. This means that for eraser bars that are at t=0 in a position $W_{\text{win}} - v_0 \cdot T \leq w_{\text{er0}} \leq W_{\text{tot}}$, the speed of the eraser bar $v_{\text{er}}$ will just stay vo, and thus $v_{\text{extra}} = 0$. In this embodiment, for the eraser bars that do get 'pushed' by the window, the eraser bar ends up at location w=$W_{\text{win}}$ at t=T. As they need to travel a distance of ($W_{\text{win}}$ - $w_{\text{er0}}$) in time T, their speed (when working with constant speeds) should be $v_{\text{er}} = (W_{\text{win}} - w_{\text{er0}}) / T$, and thus: $v_{\text{extra}} = (W_{\text{win}} - w_{\text{er0}}) / T - v_0$.

Now also for this third exemplary embodiment, we have equations for $v_{\text{extra}}$ for any starting position $w_{\text{er0}}$.

As a result, it is known, for all three embodiments, at which place the eraser bar should be at which time during the insertion of a window. The rest of the data (i.e. the older data that still fit on the screen) get "pasted" like strip, starting at the eraser bar, towards the left up to the right side of the additional window, with the rest from the right side of the display area up to the eraser bar.

After the additional window has fully entered the display area (i.e. for t>T), in some instances the eraser bar will continue with speed $v_0$ and will move until w=$W_{\text{tot}}$ and then starting back at w=$W_{\text{win}}$, moving again till w=Wtothas been reached, etc. In another example, the eraser bar may continue with speed $v_0 + v_{\text{extra}}$ for times t>T, for instance until the eraser bar reaches w=$W_{\text{tot}}$, and for times after this move with speed $v_0$. This may assist with easy continuous interpretation of the display.

Case: $v_{\text{win}} = \infty$

The same equations cannot simply be used for the case where $v_{\text{win}} = \infty$ (i.e. the window is added to the screen instantly), as this would lead to $v_{\text{extra}} = \infty$, and it should be defined what is meant with that, namely where the eraser bar ends up after it has been moved with infinite speed.

One could use the same reasoning as in the first and second exemplary embodiments above: the eraser bar gets moved to $W_{\text{win}}$ when $w_{\text{er0}} = 0$ and it does not get moved $w_{\text{er0}} = W_{\text{tot}}$, while for $0 < w_{\text{er0}} < W_{\text{tot}}$ the width over which the eraser bar is moved scales with its position. Hence, for any location of $w_{\text{er0}}$, the location $w_{\text{er}}$ where the eraser bar is moved to at the moment the window is added is given by

$$w_{\text{er}} = ((W_{\text{tot}} - W_{\text{win}})/W_{\text{tot}}) \cdot w_{\text{er0}} + W_{\text{win}}$$

Alternatively, one could use the same reasoning as in the third exemplary embodiment above: only eraser bars that were in area $0 \leq w_{\text{er0}} < W_{\text{win}}$ get moved, namely to $w = W_{\text{win}}$. Eraser bars in area $W_{\text{win}} \leq w_{\text{er0}} \leq W_{\text{tot}}$ do not get moved (i.e. $v_{\text{er}}$ stays $v_0$ and thus $v_{\text{extra}} = 0$).

Example Cases II - Left window retracting

After a window is in the display area, at the left side, it can be removed. This can be done with speed $0 < v_{\text{win}} \leq \infty$, where in this case, the velocity has a direction towards the left. As the place of the eraser bar can never be overwritten in this case, it is fine to just let it keep moving at speed $v_0$ (and thus $v_{\text{extra}} = 0$). Again, the data will be drawn back from the eraser bar as if it were a strip that extends back to the left until the right side of the window (wherever it may be at that point in time, for $0 \leq t < T$) and then further from the right side of the screen till the eraser bar. This means that if $v_{\text{win}} > $ vo, there will be older data on the screen at t=T than there were at t=0 (and these are continuously added during 0<t<T).

Of course, other embodiments possible, e.g. where the eraser bar seems to get pulled backwards by the retracting window (i.e. $v_{\text{extra}} < 0$), but the embodiment where the speed of the eraser bar stays $v_0$ may be preferable.

Example Cases III -Window entering from the right

When a window is entering from the right, after the full insertion, $0 \leq w \leq W_{\text{tot}} - W_{\text{win}}$ is the remaining available space for the waveform. Eraser bars that were at location $W_{\text{tot}} - W_{\text{win}} - v_0 \cdot T < w_{\text{er0}} \leq W_{\text{tot}}$ at t=0 would get impacted by this.

In a first exemplary embodiment, $v_{\text{extra}}$ is just chosen to be 0. Hence, the eraser bar moves with speed $v_0$ to the right and as soon as it 'hits' the window that is coming in, the eraser bar starts at the complete left of the display area (w=0) and continues with speed $v_0$. In the extreme case where $v_{\text{win}} = \infty$, this means that any eraser bar with location $W_{\text{tot}} - W_{\text{win}} < w_{\text{er0}} \leq W_{\text{tot}}$ will start at w=0 when the additional window is added.

In a second exemplary embodiment, it is prevented that the eraser bar reaches the end of the screen (i.e. the place where the additional window is at that point in time) during the insertion of the window. The system shows the eraser bar as if it is being pushed back by the entering window. Hence $v_{\text{extra}} < 0$. For example, it can be chosen such that for $W_{\text{tot}} - W_{\text{win}} - v_0 \cdot T < w_{\text{er0}} \leq W_{\text{tot}}$ the speed of the eraser bar is made equal to the speed of the entering window (and both in the direction towards the left) and thus $v_{extra}$ = -$v_0$ - $v_{win}$. On the other hand, eraser bars that were at or close to the left side of the display area should not get pushed off the display area at the left side. Therefore, the magnitude of $v_{extra}$ for $w_{er0}$ = 0 (i.e. at the complete left of the screen) should be smaller than $v_0$. Preferably, $v_{extra}$ there is equal to zero. For places of the eraser bar in between (thus 0 < $w_{er0}$ < $W_{tot}$ - $W_{win}$ - $v_0 \cdot T$) a scaling can be made for $v_{extra}$. For a linear scaling this would lead to:

$$v_{extra} = (-v_0 - v_{win}) \cdot w_{er0} / (W_{tot} - W_{win} - v_0 \cdot T), \text{ for } 0 \leq w_{er0} \leq W_{tot} - W_{win} - v_0 \cdot T$$

It is negative because it is directed towards the left; in the opposite direction of the normal eraser bar speed $v_0$.

The second exemplary embodiment is ill-defined when $v_{win}$ = ∞ (i.e. when the window is instantaneously added to the display area), because $v_{extra}$ is then minus infinity and cannot be integrated over time to get the location of the eraser bar. Therefore, it is preferable to define where the eraser bar moves to when the window is being added. For example, the eraser bar will move to $w_{er0}$ - $W_{win}$ for $W_{tot}$ - $W_{win} \leq w_{er0} \leq W_{tot}$, will not move when $w_{er0}$ = 0, and will end up at a scaled location in between, i.e. w = ($W_{tot}$ - 2$W_{win}$)/($W_{tot}$ - $W_{win}$) for $0 \leq w_{er0} \leq W_{tot}$ - $W_{win}$.

Example Cases IV - Right window retracting

A window that has been placed on the right side of the display area can be removed by retracting towards the right with speed $v_{win}$. More space becomes available for the waveform, so in case $v_{win}$ > v0, older data will get in the display area than there were before t=0. Unlike when a window enters, when a window retracts, it cannot get in the space of the eraser bar. Therefore, in a first exemplary embodiment, the eraser bar just keeps moving with speed $v_0$; hence $v_{extra}$ = 0.

In a second exemplary embodiment, extra pull is given to the eraser bar, i.e. $v_{extra}$ > 0. For example, $v_{extra}$ may be made equal to $v_{win}$ for any location of $w_{er0}$, or only at the right side, while $v_{extra}$ becomes smaller when the eraser bar was more towards the left

Example Cases V- Widening/thinning of a window or multiple windows

In the previous example cases I-IV, the addition or removal of one window was considered. However, there may be multiple additional windows. The proposed concepts are equally applicable to more than one non-overlay window. In addition, the proposed concepts may be employed for cases where a window is made wider or thinner, i.e. where the size of the window is changed.

Example Cases VI - Non-constant $v_{win}$

In the above example cases, $v_{win}$ was often assumed to be a constant speed in the timeframe of t=0 until t=T. However, it could also be changing with time in this timeframe, i.e. $v_{win}$ = $v_{win}(t)$. The speed of the eraser bar should then show proportional changes in time.

Also, in the above example cases, it was assumed that $v_{win}$ was a predetermined value (i.e. fixed). However, $v_{win}$ may depend on the situation. In particular, it may depend on the place of the eraser bar at the moment the window starts entering. For example, for a window entering from the left, if $w_{er0}$ is already close to $W_{win}$, then $v_{win}$ may be taken such that $v_{er}$ can just stay $v_0$ (i.e. $v_{extra}$=0), without the window overtaking the eraser bar (thus the eraser bar has reached a location at the right of the complete window at the moment the window has just fully entered the screen). In another example, for a window entering from the right, $v_{win}$ may be taken extra fast, when the eraser bar is approaching w=$W_{tot}$ - $W_{win}$, so that the eraser bar can keep moving at speed $v_0$ and will still end up left of the window when it has fully entered.

Example Cases VII - Multiple waveforms

In the preceding example cases, reference was made to "the eraser bar" and "the waveform". This was done because the reasoning can be applied to any waveform with its eraser bar on the screen when a window is added or removed at the height of the particular waveform. However, as there may be multiple waveforms shown on a display screen, not all with the eraser bar at the same place, a system may want to take into account the other waveforms to come to the best solution.

For example, let us assume a situation where four waveforms are simultaneously displayed one above another (i.e. in four parallel rows) across the entire width of the display area of a display screen. Further, the corresponding eraser bars for the four waveforms move with 25, 25, 6.25, and 6.25 mm/s respectively (from first (top) waveform to fourth (bottom) waveform).

Let us then assume the addition of a window to the display area, wherein the newly added window is sized and positioned so that is does not cover the uppermost/top waveform (which therefore keeps on using the complete width of the display area, but it would otherwise cover the other three (lower) waveforms (that therefore subsequently only use a smaller part of the display area according to the proposed concepts).

Although the locations of the eraser bars of the first and second waveform were initially aligned prior to the addition of the window, this will not stay like that (with an exception to be explained below), because of the difference in width they are moving over after the addition of the window. Therefore, when the window was inserted into the display area, it wouldn't have made much difference if the upper eraser bar would have been moved to the same location as the second eraser bar, or if the upper eraser bar would just have been kept on moving with speed $v_0$ while the second eraser bar had temporarily an increased speed ($v_{extra} > 0$).

However, in case the window is subsequently removed, it may be desirable to align the top two eraser bars again, because from then on, they will move over the same width and thus will stay aligned. Depending on where the eraser bars are, one may be pulled to the left for example, to make this happen.

The exception where it does matter to align the eraser bar of the first and second (upper) waveforms when inserting the window is when a smart window width $W_{win}$ is used. If the width of the window is chosen such that the remaining space for the second waveform ($W_{tot} - W_{win}$) is an integer i (i=1, 2, 3, 4,..., e.g. exactly 2 or exactly 3) times as wide as the final window width $W_{win}$, then traveling i times over the full width $W_{tot}$ (which is the width available for the first (top) waveform) takes exactly as long as traveling i+1 times over width $W_{tot} - W_{win}$. Therefore, if the eraser bars of the first (top) and second waveform, while having the window in, are aligned at one point in time, then they will once every i (for the first waveform) or i+1 (for the second waveform) time of traveling from left to right, have a width $W_{tot} - W_{win}$ where the eraser bars travel together, aligned. Because having alignment of eraser bars is desirable, it is thus beneficial to have such a smart window width. In such a case, it is preferable to give the eraser bar of the first waveform, while inserting the window, the same $v_{extra}$ as the second waveform, to keep them aligned. Alternatively, at the time the window has just moved in completely, the eraser bar of the second waveform has moved to a location that is exactly the window width $W_{win}$ further to the right than the location of the eraser bar of the first waveform at that time, so that after one or more times of reaching the right end of the screen and starting back from the left, the eraser bars are aligned.

There are other possibilities to give the window a smart window width $W_{win}$. Instead of working with integers, one could work with fractions where the numerator and denominator are integers, preferably low integers (e.g. 1, 2, 3, etc.). For example, fraction 3/2, or 1.5, could be used, meaning that $W_{win}$ is chosen such that $W_{tot} - W_{win} = 1.5*W_{win}$. However, in these cases, after initial alignment, it takes longer before alignment is reached for the second time than for low values of i (in the example of $W_{tot} - W_{win} = 1.5*W_{win}$, it takes five times going from left to right of the second waveform and three times for the first waveform). In the examples, $W_{win}$ was smaller than $W_{tot} - W_{win}$, but it also works for $W_{win}$ being larger than $W_{tot} - W_{win}$, namely when $W_{win}$ is an integer (or fraction) times as wide as $W_{tot} - W_{win}$. Also in these cases, the locations of the eraser bars of the various waveforms right after insertion of a window are preferably chosen such that alignment once every couple of wraps remains.

In the paragraphs above, we focused on waveforms with eraser bars with the same speed vo, but where one of the two gets/has/had a reduced width available because of the additional window, while the other waveform always has the full width available.

Another aspect to consider is that waveforms that do not have the same eraser bar speed $v_0$ may have a different location of the eraser bar $w_{er0}$ when the window is about to get inserted or deleted. In that case, it might be desired to make the behavior of the motion of the eraser bars during insertion or deletion of a window similar for the second to fourth eraser bar. To that purpose, it may be determined where the "most critical" eraser bar is. This may for example be the eraser bar that most easily gets overtaken by the window that gets inserted if not enough extra speed is given to the eraser bar. In this case, it can be either the second eraser bar, or the third and fourth eraser bars. For that eraser bar, $v_{extra}$ can be determined by following one of the equations of the example cases above. Then the other eraser bars may be moved such that they have the exact same speed or a scaled speed.

When the different eraser bar speeds $v_0$ of the different waveforms are chosen such that there is period alignment of the eraser bars at that utmost left point of the display area (e.g. when $v_0$ of one waveform is chosen to be an integer i times $v_0$ of another waveform, with an initial alignment), then it may be beneficial that the final location of the eraser bars right after insertion or deletion of a window takes into account that this type of alignment remains to exist.

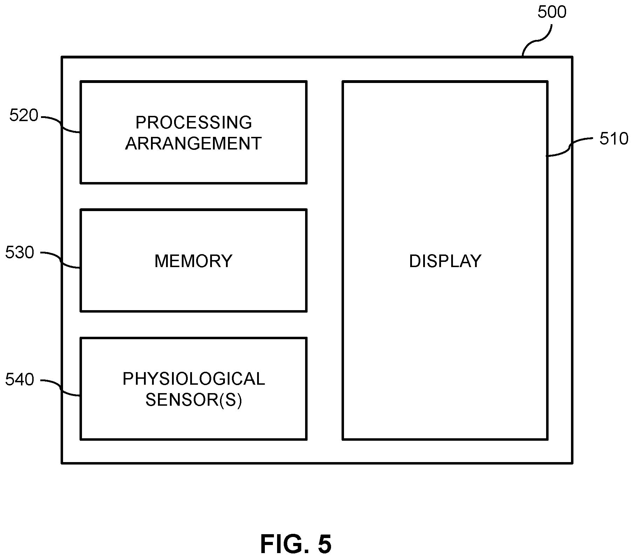

Referring now to Fig. 5, there is depicted a simplified block diagram of display system 500 for displaying data according to a proposed embodiment. The display system comprises a display 510, a processing arrangement 520, a memory 530 and one or more physiological sensors 540. The one or more physiological sensors 540 are adapted to sense variations in the value of one or more time-varying signals. The memory 530 is adapted to a computer program comprising computer program code means adapted to implement the method of a proposed embodiments (e.g. any one or more of the embodiments described above). The processing arrangement 520 is configured to run the computer program stored in the memory 520 and thus control the display 510 to display of data according to the embodiment(s) implemented by the computer program.

The display system 500 is just one example of a display system for implementing the concepts of the proposed invention. The methods 100 and 300 may equally well be implemented without the use of a processing arrangement at all but may be implemented solely in hardware. For example, some embodiments may use a field-programmable gate array (FPGA) connected to a standard display, or an LED Matrix with a small logic circuit for each LED. The waveform data that is displayed in a given pixel column of the display is controlled based on the fixed position of that pixel column relative to the moving display position of the eraser, wherein the display position of the eraser is calculated using a method of the proposed invention. This waveform data is accessed from a memory with stored wave data and used to determine the intensity of each pixel in the pixel column.

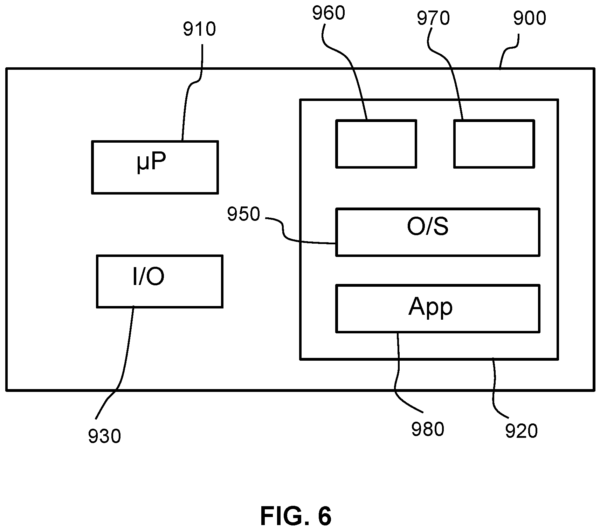

Fig. 6 illustrates an example of a computer 900 within which one or more parts of an embodiment may be employed. Various operations discussed above may utilize the capabilities of the computer 900. In this regard, it is to be understood that system functional blocks can run on a single computer or may be distributed over several computers and locations (e.g., connected via the internet).

The computer 900 includes, but is not limited to, PCs, workstations, laptops, PDAs, palm devices, servers, storages, and the like. Generally, in terms of hardware architecture, the computer 900 may include one or more processors 910, memory 920 and one or more I/O devices 930 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 910 is a hardware device for executing software that can be stored in the memory 920. The processor 910 can be virtually any custom made or commercially available processor, a central processing unit (CPU), a digital signal processor (DSP), or an auxiliary processor among several processors associated with the computer 900, and the processor 910 may be a semiconductor based microprocessor (in the form of a microchip) or a microprocessor.

The memory 920 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 920 may incorporate electronic, magnetic, optical, and/or other types of storage media. Note that the memory 920 can have a distributed architecture, where various components are situated remote from one another, but can be accessed by the processor 910.

The software in the memory 920 may include one or more separate programs, each of which comprises an ordered listing of executable instructions for implementing logical functions. The software in the memory 920 includes a suitable operating system (O/S) 950, compiler 960, source code 970, and one or more applications 980 in accordance with exemplary embodiments. As illustrated, the application 980 comprises numerous functional components for implementing the features and operations of the exemplary embodiments. The application 980 of the computer 900 may represent various applications, computational units, logic, functional units, processes, operations, virtual entities, and/or modules in accordance with exemplary embodiments, but the application 980 is not meant to be a limitation.

The operating system 950 controls the execution of other computer programs, and provides scheduling, input-output control, file and data management, memory management, and communication control and related services. It is contemplated by the inventors that the application 980 for implementing exemplary embodiments may be applicable on all commercially available operating systems.

Application 980 may be a source program, executable program (object code), script, or any other entity comprising a set of instructions to be performed. When a source program, then the program is usually translated via a compiler (such as the compiler 960), assembler, interpreter, or the like, which may or may not be included within the memory 920, so as to operate properly in connection with the O/S 950. Furthermore, the application 980 can be written as an object oriented programming language, which has classes of data and methods, or a procedure programming language, which has routines, subroutines, and/or functions, for example but not limited to, C, C++, C#, Pascal, Python, BASIC, API calls, HTML, XHTML, XML, ASP scripts, JavaScript, FORTRAN, COBOL, Perl, Java, ADA, .NET, and the like.

The I/O devices 930 may include input devices such as, for example but not limited to, a mouse, keyboard, scanner, microphone, camera, etc. Furthermore, the I/O devices 930 may also include output devices, for example but not limited to a printer, display, etc. Finally, the I/O devices 930 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 930 also include components for communicating over various networks, such as the Internet or intranet.

If the computer 900 is a PC, workstation, intelligent device or the like, the software in the memory 920 may further include a basic input output system (BIOS) (omitted for simplicity). The BIOS is a set of essential software routines that initialize and test hardware at start-up, start the O/S 950, and support the transfer of data among the hardware devices. The BIOS is stored in some type of read-only-memory, such as ROM, PROM, EPROM, EEPROM or the like, so that the BIOS can be executed when the computer 900 is activated.

When the computer 900 is in operation, the processor 910 is configured to execute software stored within the memory 920, to communicate data to and from the memory 920, and to generally control operations of the computer 900 pursuant to the software. The application 980 and the O/S 950 are read, in whole or in part, by the processor 910, perhaps buffered within the processor 910, and then executed.

When the application 980 is implemented in software it should be noted that the application 980 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 980 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The methods of Figs 1 and 3, and the system of Fig. 5 may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagrams of Figs 5 and 6 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems.

The flowcharts and block diagrams in the Figures illustrate the architecture, functionality, and operation of possible implementations of systems, methods, and computer program products according to various embodiments of the present invention. In this regard, each block in the flowcharts or block diagrams may represent a module, segment, or portion of instructions, which comprises one or more executable instructions for implementing the specified logical function(s). In some alternative implementations, the functions noted in the block may occur out of the order noted in the Figures. For example, two blocks shown in succession may, in fact, be executed substantially concurrently, or the blocks may sometimes be executed in the reverse order, depending upon the functionality involved. It will also be noted that each block of the block diagrams and/or flowchart illustrations, and combinations of blocks in the block diagrams and/or flowchart illustrations, can be implemented by special purpose hardware-based systems that perform the specified functions or acts or carry out combinations of special purpose hardware and computer instructions, the architecture, functionality, and operation of possible implementations of systems, methods, and computer program products according to various embodiments of the present invention.

Variations to the disclosed embodiments can be understood and effected by those skilled in the art in practicing the claimed invention from a study of the drawings the disclosure, and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. If the term "adapted to" is used in the claims or description it is noted the term "adapted to" is intended to be equivalent to the term "configured to". Any reference signs in the claims should not be construed as limiting the scope.

**Claims**

1. A method (100) for controlling a display of data, the method comprising:
generating (110) a first display control signal for controlling a display (200) in a first time interval, the first display control signal describing:

> the display of a waveform (200) in a display area of the display, the waveform visually indicating the variation in the value of a time-varying signal over a first time period; and
> the display of a first eraser bar (220) in the display area, the display position of the first eraser bar in the display area evolving over time to indicate the evolution in a time origin of the first time period with time such that at any given time the difference between the display position of the first eraser bar and the display position of a point in the waveform indicates the difference between the time origin of the first time period and a capture time associated with the point in the waveform; and
> generating (120) a second display control signal for controlling the display in a second time interval that does not overlap with the first time interval, the second display control signal describing:

>> the display of a window (230) in a first portion of the display area, the display area further comprising a second portion in which the window is not displayed;
>> the display of a truncated version of the waveform (240) in the second portion of the display area, wherein the truncated version of the waveform visually indicates the variation in the time-varying signal over a second time period that is shorter than the first time period and at least partially overlaps with the first time period; and
>> the display of a second eraser bar (250) in the second portion of the display area, the display position of the second eraser bar in the second portion of the display area evolving over time to indicate the evolution in a time origin of the second time period with time such that at any given time the difference between the display position of the second eraser bar and the display position of a point in the truncated version of the waveform indicates the difference between the time origin of the second time period and a capture time associated with the point in the truncated version of the waveform,
>> wherein the evolution in the position of the second eraser bar over time is controlled based on the evolution of the display position of the first eraser bar over time.

2. The method of claim 1, wherein the second display control signal further describes the display of the window entering the first portion of the display area at a finite window entry speed $v_{win}$ over a window entry time period within the second time interval, and
wherein the evolution of the display position of the second eraser bar over time is further controlled based on the value of the window entry speed.

3. The method of claim 2, wherein controlling the evolution in the display position of the second eraser bar over time comprises controlling a speed at which the second eraser bar moves across the display over the window entry time period $v_{er2}$ to be equal to the sum of the speed of the first eraser bar at the end of the first time interval $v_{er1}$ and an additional speed, and
wherein the value of the additional speed is determined based on the value of the window entry speed and the display position of the second eraser bar.

4. The method of claim 3, wherein the additional speed is equal to a scale factor multiplied by the window entry speed, the scale factor being greater than or equal to zero and less than or equal to 1, and
wherein the value of the scale factor varies as a function of the display position of the second eraser bar.

5. The method of any of claims 2-4, wherein second display control signal controls the window entry speed of the window based on at least one of:

> a display position of the first eraser bar at the end of the first time interval; and
> a speed at which the display position of the first eraser bar changes over time at the end of the first time interval.

6. The method of any preceding claim, wherein controlling the evolution in the display position of the second eraser bar over time comprises controlling an initial display position of the second eraser bar at the start of the second time interval comprising:

> controlling the initial display position of the second eraser bar to have a first value if the display position of the first eraser bar at the end of the first time interval is in the first portion of the display area; and
controlling the initial display position of the eraser bar to have a second value if the display position of the second eraser bar at the end of the first time interval is in the second portion of the display area.

7. The method of claim 6, wherein controlling the initial display position of the second eraser bar to have a second value comprises controlling the initial display position of the second eraser bar to be the same as the display position of the first eraser bar at the end of the first time interval.

8. A method (300) for controlling a display of data, the method comprising:
generating (310) a first display control signal for controlling a display (400) in a first time interval, the first display control signal describing:

the display of a window (430) in a first portion of a display area, the display area further comprising a second portion in which the window is not displayed;
the display of a waveform (410) in the second portion of the display area, the waveform visually indicating the variation in a time-varying signal over a first time period; and
the display of a first eraser bar (420) in the second portion of the display area, the display position of the first eraser bar in the second portion of the display area evolving over time to indicate the evolution in a time origin of the first time period with time such that at any given time the difference between the display position of the first eraser bar and the display position of a point in the waveform indicates the difference between the time origin of the first time period and a capture time associated with the point in the waveform; and
generating (320) a second display control signal for controlling a display in a second time interval that does not overlap with the first time interval, the second display control signal describing:

the display of an elongated version of the waveform (440) in the display area, wherein the elongated version of the waveform visually indicates the variation in the time-varying signal over a second time period that is longer than the first time period and at least partially overlaps with the first time period; and
the display of a second eraser bar (450) in the display area, the display position of the second eraser bar in the display area evolving over time to indicate the evolution in a time origin of the second time period with time such that at any given time the difference between the display position of the second eraser bar and the display position of a point in the elongated version of the waveform indicates the difference between the time origin of the second time period and a capture time associated with the point in the elongated version of the waveform,
wherein the evolution of the position of the second eraser bar over time is controlled based on the evolution of the display position of the first eraser bar over time.

9. The method of claim 8, wherein the first display control signal further describes the display of the window retracting from the first portion of the display area at a finite window exit speed over a window exit time period within the first time interval, and
wherein the evolution of the display position of the first eraser bar during the window exit time period is controlled based on the value of the window exit speed.

10. The method of claim 9, wherein controlling the evolution of the display position of the first eraser bar during the window exit time period comprises controlling the speed at which the display position the first eraser bar changes over time based on the value of the window exit speed and the display position of the first eraser bar.

11. The method of any preceding claim, wherein controlling the evolution in the display position of the second eraser bar over time comprises controlling the evolution in the display position of the second eraser bar based on at least one of:

a display position of the first eraser bar at the end of the first time interval; and
a speed at which the display position of the first eraser bar changes over time at the end of the first time interval.

12. The method of any preceding claim, wherein controlling the evolution in the position of the second eraser bar over time comprises controlling at least one of:

an initial display position of the second eraser bar at the start of the second time interval; and
an initial speed at which the display position of the second eraser bar changes over time at the start of the second time interval.

**13.** A computer program comprising computer program code means adapted, when said computer program is run on a computer, to implement the method of any of claims 1-12.

**14.** A processing arrangement (520) for controlling a display of data configured to run the computer program of claim 13.

**15.** A display system (500) for displaying data, the display system comprising:

a display (510); and
the processing arrangement (520) of claim 14 for controlling the display.

110

GENERATE FIRST DISPLAY CONTROL SIGNAL
DESCRIBING THE DISPLAY OF A WAVEFORM AND A
FIRST ERASER BAR

120

GENERATE SECOND DISPLAY CONTROL SIGNAL
DESCRIBING THE DISPLAY  OF A WINDOW, A
TRUNCATED VERSION OF THE WAVEFORM, AND A
SECOND ERASER BAR

100

**FIG. 1**

FIG. 2A

FIG. 2B

FIG. 2C

310

GENERATE FIRST DISPLAY CONTROL SIGNAL
DESCRIBING THE DISPLAY OF A WINDOW, A
WAVEFORM AND A FIRST ERASER BAR

320

GENERATE SECOND DISPLAY CONTROL SIGNAL
DESCRIBING THE DISPLAY OF AN ELONGATED
VERSION OF THE WAVEFORM AND A SECOND
ERASER BAR

300

# FIG. 3

400 440 $V_{er1}$ 410

430

420 415

**FIG. 4A**

400 450 $V_{er2}$

430'

**FIG. 4B**

500

520 —— PROCESSING
ARRANGEMENT

—— 510

530 —— MEMORY

DISPLAY

540 —— PHYSIOLOGICAL
SENSOR(S)

**FIG. 5**

**FIG. 6**

Europäisches
Patentamt

European
Patent Office

Office européen
des brevets

## EUROPEAN SEARCH REPORT

**Application Number**

EP 24 21 1081

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 00/42913 A1 (GE MARQUETTE MEDICAL SYS INC [US]) 27 July 2000 (2000-07-27) * figure 2 * * second paragraph; page 1 * * second paragraph; page 5 * ----- | 1-15 | INV. A61B5/00 |
| A | US 2013/044111 A1 (VANGILDER JAMES [US] ET AL) 21 February 2013 (2013-02-21) * figure 7 * ----- | 1-15 | |

**TECHNICAL FIELDS SEARCHED (IPC)**

A61B

The present search report has been drawn up for all claims

| Place of search | Date of completion of the search | Examiner |
|---|---|---|
| The Hague | 19 June 2025 | Albrecht, Ronald |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
    ..................................................................
& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 24 21 1081

This annex lists the patent family members relating to the patent documents cited in the above-mentioned European search report.
The members are as contained in the European Patent Office EDP file on
The European Patent Office is in no way liable for these particulars which are merely given for the purpose of information.

19-06-2025

| Patent document cited in search report | | Publication date | Patent family member(s) | | Publication date |
|---|---|---|---|---|---|
| WO 0042913 | A1 | 27-07-2000 | EP | 1065972 A1 | 10-01-2001 |
| | | | JP | 2002535028 A | 22-10-2002 |
| | | | WO | 0042913 A1 | 27-07-2000 |
| US 2013044111 | A1 | 21-02-2013 | AU | 2012255897 B2 | 17-11-2016 |
| | | | BR | 112013029165 A2 | 31-01-2017 |
| | | | CA | 2835937 A1 | 22-11-2012 |
| | | | CN | 103648372 A | 19-03-2014 |
| | | | EP | 2709518 A1 | 26-03-2014 |
| | | | GB | 2505133 A | 19-02-2014 |
| | | | JP | 6235461 B2 | 22-11-2017 |
| | | | JP | 2014518715 A | 07-08-2014 |
| | | | KR | 20140045359 A | 16-04-2014 |
| | | | MX | 337609 B | 10-03-2016 |
| | | | US | 2013044111 A1 | 21-02-2013 |
| | | | WO | 2012158720 A1 | 22-11-2012 |

EPO FORM P0459

For more details about this annex : see Official Journal of the European Patent Office, No. 12/82